(19) Europäisches Patentamt
European Patent Office
Office européen des brevets

(11) **EP 0 936 467 B1**

(12) **EUROPEAN PATENT SPECIFICATION**

(45) Date of publication and mention
of the grant of the patent:
**18.04.2007 Bulletin 2007/16**

(51) Int Cl.:
***G01N 33/00*** *(2006.01)*

(21) Application number: **99102680.8**

(22) Date of filing: **12.02.1999**

(54) **Exhaust gas analyzer and modal mass analysis method by gas trace process using the analyzer thereof**

Auspuffgasanalysevorrichtung und modales Massenanalyseverfahren durch Gasspurverfahren unter Benutzung dieser Analysevorrichtung

Analyseur de gaz d'échappement et méthode d'analyse de masse modale par un processus de traces de gaz en utilisant cet analyseur

(84) Designated Contracting States:
**DE GB**

(30) Priority: **13.02.1998 JP 4888498**

(43) Date of publication of application:
**18.08.1999 Bulletin 1999/33**

(73) Proprietor: **HORIBA, LTD.**
**Minami-ku**
**Kyoto (JP)**

(72) Inventors:
• **Adachi, Masayuki**
**c/o Horiba, Ltd**
**Kyoto (JP)**

• **Hirano, Takashi**
**c/o Horiba, Ltd**
**Kyoto (JP)**

(74) Representative: **Müller - Hoffmann & Partner**
**Patentanwälte,**
**Innere Wiener Strasse 17**
**81667 München (DE)**

(56) References cited:
**EP-A- 0 222 994      EP-A- 0 230 673**
**EP-A- 0 855 578      US-A- 4 121 455**
**US-A- 5 639 957**

• **PATENT ABSTRACTS OF JAPAN vol. 010, no. 089 (P-444), 8 April 1986 (1986-04-08) & JP 60 225029 A (MAZDA KK), 9 November 1985 (1985-11-09)**

**Description**

The present invention relates to an exhaust gas analyzer for measuring the mass of each specific component gas in the exhaust gas emitted from automobiles by driving modes and to a modal mass analysis method by the gas trace process using the analyzer thereof.

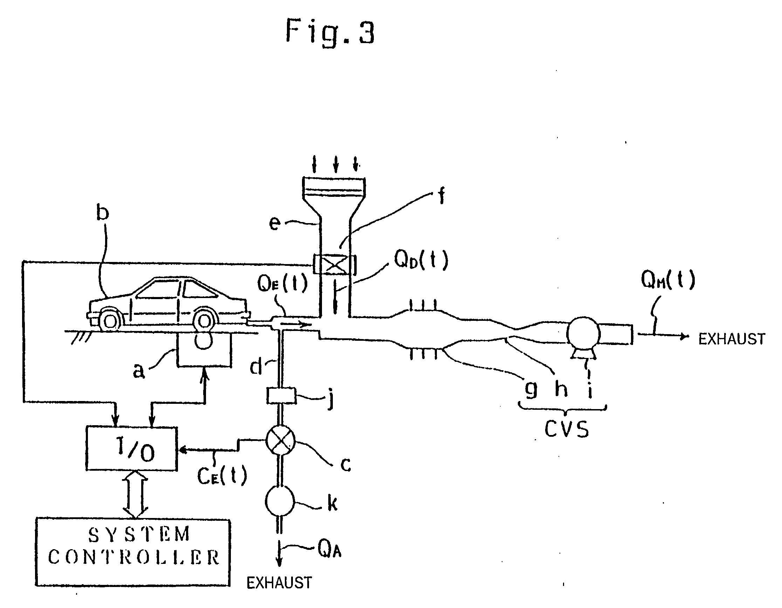

Because the components of exhaust gas emitted from vehicle such as automobiles, etc. vary to driving modes, the exhaust gas flow rate of each specific component must be measured in real time in accordance with each driving mode, and conventionally, for example, as shown in Fig. 3, a dilution analysis process has been adopted in which the exhaust gas in each driving mode is diluted to have a specified constant flow rate by atmosphere, introduced into a gas component analyzer c, and the emitted rate of each specific component is determined.

As a method for determining the emitted rate of the specific component, a modal mass analysis method using a dilution analysis process is known. In this analysis method, let the flow rate of the exhaust gas sucked into a sampling passage d for concentration measurement be $Q_A$ (constant), dilution air rate measured by ultrasonic flow meter f at a dilution air inflow passage e be $Q_D(t)$, a total suction flow rate by the constant volume sampler CVS be $Q_M$ (constant); then, the exhaust gas flow rate (containing moisture) emitted from the specimen vehicle b $Q_{WE}(t)$ can be found from an arithmetic expression comprised of

$$Q_{WE}(t) = Q_A + Q_M - Q_D(t) \qquad \qquad ...(3)$$

On the other hand, let the concentration of the components to be measured in the exhaust gas measured by the gas concentration analyzer c be $C_{WE}(t)$; then, the exhaust volume (mass) M(t) of the components to be measured in the exhaust gas can be found by an arithmetic expression comprised of

$$M(t) = \rho \times C_{WE}(t) \times Q_{WE}(t) \qquad \qquad ...(4)$$

by each driving mode. This kind of arithmetic method composed of Eq. (3) and Eq. (4) is the modal mass analysis method applied to the dilution analysis process. In Fig. 3, reference character g designates a heat exchanger, h a constant flow-rate venturi pipe, i a suction pump, j a dehumidifier, and k a suction pump.

Consequently, in the modal mass analysis method by the dilution analysis process described above, when a component which is the same as that to be measured in the exhaust gas is present in the atmosphere, there is a possibility in that the measurement results are subject to an influence, and there is a difficulty to generate a limit in the measuring accuracy.

Even if a large-flow-rate air purifier is used for dilution, it is obviously disadvantageous for the analyzer to analyze by further diluting the measured component if the measured component is at low concentration. In addition, a great amount of investment is required for the equipment for purifying air or the CVS (constant-volume sampler) equipment for sucking diluting air at a constant flow rate, resulting in high cost for the overall analysis equipment as well as an increase size.

On the other hand, in the computation of Eq. (4) for determining the exhaust rate M(t) of the component to be measured for each driving mode, the value containing the moisture, that is, the wet-base value, is used for the concentration $C_{WE}(t)$, but because of water interference of the gas analyzer c, the exhaust gas with moisture removed by the dehumidifier j is introduced, the concentration $C_{WE}(t)$ is unable to be directly detected by the gas analyzer c.

Therefore, for convenience, the dry-base (free of moisture) concentration $C_{DE}(t)$ detected by the gas analyzer c is converted into the wet-base concentration $C_{WE}(t)$ separately, and charged in Eq. (4). For example, let the moisture content (= moisture content removed by the dehumidifier j) contained in the exhaust gas be $C_{H20}(t)$, the wet-base concentration $C_{WE}(t)$ can be determined by the conversion equation comprised of

$$C_{WE}(t) = C_{DE}(t) \times (1 - C_{H2o}(t)) \qquad \qquad ...(5)$$

The moisture content $C_{H20}(t)$ is $C_{H20}(t) = 1/10 (= 0.1)$ when the flow rate $Q_{WE}(t)$ of the exhaust gas is supposed to be 1, for example, when 10 % moisture is contained in the total exhaust gas.

Consequently, the value of the moisture content $C_{H20}(t)$ is unable to be actually measured, and a value empirically considered adequate (assumed value) is used, but because the actual moisture content $C_{H20}(t)$ varies according to fuels and measurement conditions (seasons), there are cases in which the wet-base concentration $C_{WE}(t)$ determined by Eq. (5) differs from the actual value and as a result, the value of the exhaust rate $M(t)$ of the component to be measured which is determined by Eq. (4) cannot be said accurate, generating difficulty in reproducibility.

Another gas analyzer for the simultaneous measurement of a plurality of components contained in a sample gas is known from EP 0 222 994. According to said document, there is provided sample gas flow-dividing means in a sample gas-introducing passage for dividing a flow of the sample gas at an appointed ratio. The sample gas flow-dividing means further comprises a plurality of gas concentration detectors connected therewith in parallel. Sample gas-diluting means are provided between the sample gas flow-dividing means and said gas concentration detectors for diluting the sample gas at adjustable ratios. The above-mentioned accuracy problems also apply to the technique described in that document.

EP A 0 855 578 which was published after the priority date of the present application discloses another exhaust gas flow measuring equipment of internal combustion engines.

EP A 0 230 675 discloses another modal mass analysis method for exhaust gases from motor cars. Also the method according to that document uses the dilution-quantity method in order to determine the concentration of certain gases in an exhaust gas.

Under these circumstances, it is the main object of this invention to provide an exhaust gas analyzer which can accurately measure the exhaust rate of each of specific component gas in the automobile exhaust gas by driving modes in real time without using diluting air and a method for computing the exhaust rate thereof.

In order to achieve the above described problems to be solved, the present invention provides an exhaust gas analyzer as defined in claim 1 and a modal mass analysis method as defined in claim 3.

That is to say, the invention set forth in claim 1 is characterized in that a trace gas supply source, a flow rate controller for adjusting the supply rate of the trace gas to a vehicle, a dehumidifier mounted to the passage of the exhaust gas from the vehicle, a trace gas detector and a gas analyzer for measuring the component to be measured in the exhaust gas, both of which are installed in parallel downstream the dehumidifier in the passage.

The invention set forth in claim 2 is characterized by an exhaust gas analyzer comprising an exhaust gas analyzer as stated in claim 1, wherein a means for computation is incorporated for determining the exhaust volume (mass) $M(t)$ of the components to be measured by driving modes in accordance with the computation program by means of the modal mass analysis method using the gas trace process set and stored in memory in advance.

The invention set forth in claim 3 is characterized by a modal mass analysis method comprising the exhaust gas analyzer as stated in claim 1 or claim 2, wherein a gas trace process is incorporated to find the dry based exhaust gas flow rate $Q_{DE}(t)$ from the known flow rate $Q_H(t)$ of trace gas mixed in the exhaust gas emitted from the vehicle offered for driving simulation test based on the specified driving mode change-over sequence and the concentration $C_H(t)$ of trace gas measured at a specified sampling time using the arithmetic expression comprised of:

$$Q_{DE}(t) \; = \; Q_H(t) \; \div \; C_H(t) \qquad\qquad ...(1)$$

and at the same time to measure the concentration $C_{DE}(t)$ of the component to be measured in the exhaust gas in the specified sampling time which is the same as that for the measurement of the trace gas concentration $C_H(t)$, and to determine the exhaust volume (mass) $M(t)$ of the component to be measured by driving modes by the arithmetic expression comprised of:

$$M(t) \; = \; \rho \; x \; C_{DE}(t) \; x \; Q_{DE}(t) \qquad\qquad ...(2)$$

(where, $\rho$ is density of the component to be measured)

The modal mass analysis method by trace gas using this equipment uses no dilution air, and therefore, is free from any influence of specific components contained in diluting air, needs no complicated compensation for temperature or pressure of the exhaust gas, and is scarcely subject to pulsation of exhaust gas.

Because the trace gas detector and gas analyzer are arranged in parallel, it is possible to introduce exhaust gas into both apparatus at the same timing, and because it is possible to determine the dry based flow rate $Q_{DE}(t)$ of the exhaust gas measured by the trace gas detector and the concentration $C_{DE}(t)$ of the component to be measured by the gas analyzer at the same timing, there is no need for adjusting time for both apparatus.

In addition, there is an advantage in that the exhaust gas can be sampled from an optional position after the combustion chamber when the trace gas is injected from the suction side of the engine, and from an optional position with a suitable distance provided from the injection point when the trace gas is injected into the tail pipe, and the degree of freedom in the equipment layout can be markedly improved. The elimination of CVS equipment and air purifier further increases the degree of freedom, and it is possible to provide the equipment in a compact size and at a low cost. In addition, because the CVS equipment is not used, it has an advantage of requiring no purified air, achieving great reduction in running cost.

In the modal mass analysis method using trace gas by the equipment, because it is possible to actually measure the dry-base flow rate $Q_{DE}(t)$ that can be directly multiplied by the dry-base concentration $C_{DE}(t)$ (see Eq. (1)), it is possible to find the highly accurate measured value at high reliability without using the conventional assumed value of the moisture content in the computation process in Eq. (2).

By the way, in this method, it is possible to directly find the wet-base exhaust rate M(t) of the component to be measured by the dry-base computation in Eq. (2). The principle is described as follows. First, the following relational expression holds between the wet-base concentration $C_{WE}(t)$ and dry-base concentration $C_{DE}(t)$.

$$C_{WE}(t) = C_{DE}(t) \times (1 - C_{H20}(t)) \qquad \ldots (6)$$

And between the flow rate of wet-base exhaust gas $Q_{WE}(t)$ and the flow rate of dry-base exhaust gas $Q_{DE}(t)$, the following relational expression holds for:

$$Q_{WE}(t) = Q_{DE}(t) \times 1/(1 - C_{H20}(t)) \qquad \ldots (7)$$

Therefore, substitution of values of $C_{DE}(t)$ and $Q_{DE}(t)$ found from Eq. (6), (7) in Eq. (2) gives Eq. (4).

$$
\begin{aligned}
M(t) &= \rho \times C_{DE}(t) \times Q_{DE}(t) \qquad \ldots (2) \\
&= \rho \times \{C_{WE}(t)/(1 - C_{H20}(t)\} \times \{Q_{WE}(t) \times (1 - C_{H20}(t)\} \\
&= \rho \times C_{WE}(t) \times Q_{WE}(t)
\end{aligned}
$$

That is, the computation equation (2) by the dry base is equal to the computation equation (4) by the conventional wet base. Because at this point, the moisture content $C_{H20}$ which is unable to be actually measured is cancelled, as a result, causes of errors generated by assumption can be eliminated. Consequently, with this method, it is possible to find the measured value at higher accuracy and more improved reliability than with the modal mass analysis method using the conventional dilution analysis process.

Further details, features and advantages of the invention are apparent from the following detailed description, wherein:

Fig. 1 schematically shows a basic embodiment of the exhaust gas analyzer according to the invention;

Fig. 2 is a block diagram showing a preferred embodiment of the exhaust gas analyzer; and

Fig. 3 is a block diagram showing one example of the equipment for embodying the conventional modal mass analysis method.

Referring now to drawings, the embodiments of the exhaust gas analyzer according to the invention and of modal mass analysis method using trace gas will be described in detail hereinafter.

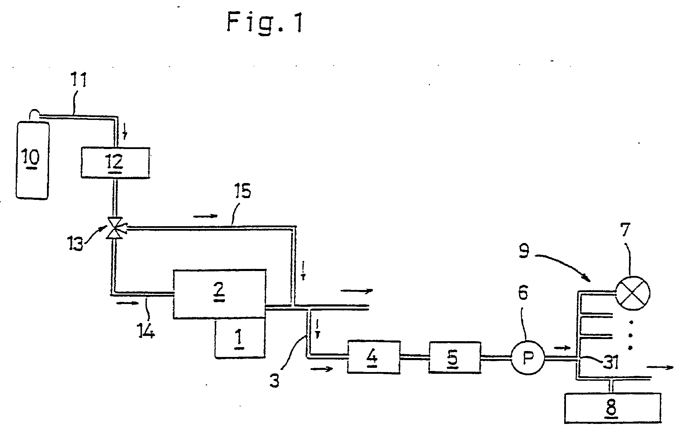

Fig. 1 shows a basic configuration of the equipment. Numeral 1 designates a chassis dynamo, numeral 2 the specimen vehicle, numeral 3 an exhaust gas sampling pipe connected to a tail pipe, numeral 4 a filter, numeral 5 a dehumidifier, numeral 6 a suction pump, numeral 7 a gas analyzer using either NDIR process or FTIR process, etc. for measuring the component to be measured, and numeral 8 a trace gas detector (helium detector) connected to the gas analyzer 7 in parallel via the branching point 31 of the exhaust gas introducing pipe 3, and the analyzer 9 comprises these gas analyzer 7 and trace gas detector 8.

Numeral 10 designates the He gas cylinder (trace gas supply source) for supplying He gas as trace gas, numeral 11 a trace gas supply pipe, numeral 12 a mass flow controller as a flow rate controller, numeral 13 a three-way valve (for example, electromagnetic three-way selector valve), numeral 14 a branch supply pipe to be connected to the suction side of the engine of the specimen vehicle 2, and numeral 15 a branch supply pipe to be connected to the exhaust gas introducing pipe 3.

Because the modal mass analysis method by the gas trace method using He gas with simple construction can be embodied with the equipment configured in this way, the equipment does not have to consider the influence of the existence of the component in the atmosphere same as that measured in exhaust gas, does not require any complicated compensation for temperature or pressure of the exhaust gas, and becomes scarcely subject to pulsation of exhaust gas.

Because the trace gas detector 8 and gas analyzer 7 are arranged in parallel, it is possible to introduce exhaust gas in same timing into both apparatus, and the dry based exhaust gas flow rate $Q_{DE}(t)$ measured by the trace gas analyzer 8 and the concentration $C_{DE}(t)$ of the component measured by the gas analyzer 7 can be determined in the same timing, and therefore, time adjustment is no longer required for both apparatus.

In addition, there is an advantage in that the exhaust gas can be sampled from an optional position after the combustion chamber when the He gas is injected from the suction side of the engine, and from an optional position with a suitable distance provided from the injection point when the He gas is injected into the tail pipe, and the degree of freedom in the equipment layout can be markedly improved. Because the elimination of CVS equipment and air purifier further increases the degree of freedom, and it is possible to provide the equipment in a compact size and at a low cost. In addition, because the CVS equipment is not used, it has an advantage of requiring no purified air, achieving great reduction in running cost.

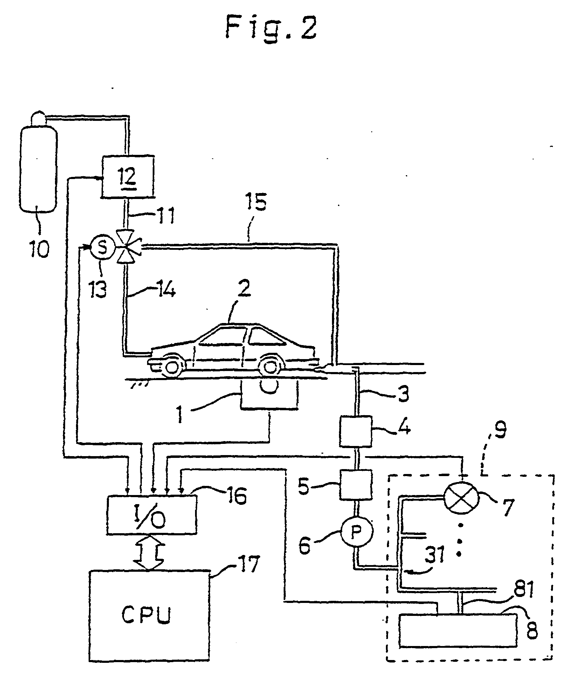

Fig. 2 shows a preferred embodiment of the equipment, wherein the chassis dynamo 1, gas analyzer 7, trace gas detector 8, mass flow controller 12, and electromagnetic three-way valve 13 are connected to the system controller (CPU) 17 containing a means for computation via an I/O interface 15, and in accordance with the computation program set and stored in memory in advance, by the modal mass analysis method using the gas trace process, the exhaust volume (mass) M(t) of the specific component gas can be determined in real time in accord with the driving pattern. To measure multiple components simultaneously, a plurality of gas analyzers are connected in parallel if the gas analyzer 7 is based on the NDIR method, but when the FTIR method is used, multiple components can be simultaneously and continuously measured with only one single gas analyzer 7.

In this equipment, 100 % He gas is introduced from the branch supply pipe 13 to the suction port side of the engine or from the branch supply pipe 14 to the tail pipe in order to thoroughly mix He gas in the exhaust gas after the flow rate is adjusted by the mass flow controller 12. The exhaust gas sampled from downstream the tail pipe by the exhaust gas sampling tube 3 as sample gas is introduced to the analyzer 9 via the filter 4 and the dehumidifier 5, and by this, the gas analyzer 7 and the trace gas detector 8 can be protected from steam or contaminant particles, etc. Numeral 81 is a capillary or film which allows only He gas to pass.

The driving mode of the vehicle 2 can be continuously changed over in the specified sequence by the chassis dynamo 1 controlled and driven by the command from the system controller 17, and during this period, detection signals from the gas analyzer 7 and the trace gas detector 8 are imputed to CPU17, and the exhaust volume (mass) M(t) of the component to be measured can be determined in real time by driving mode by the following computations in accordance with the computation program set and stored in memory in advance.

First, the concentration $C_H(t)$ of trace gas is measured in the constant sampling time by the trace gas detector 8, and from the concentration $C_H(t)$ and the known flow rate $Q_H(t)$ of He gas, the dry-base exhaust gas flow rate $Q_{DE}(t)$ is found from the arithmetic expression comprised of

$$Q_{DE}(t) = Q_H(t) \div C_H(t) \qquad\qquad ...(1)$$

(where the units used in Eq. (1) are cc/min for $Q_H(t)$, ppm for $C_H(t)$, and $m^3$/min for $Q_{DE}(t)$)

while the dry-base concentration $C_{DE}(t)$ of the component to be measured in the exhaust gas is measured by the gas analyzer 7 in the same sampling time as in the case of measurement of the He gas, and by the arithmetic expression of

$$M(t) = \rho \times C_{DE}(t) \times Q_{DE}(t) \qquad\qquad ...(2)$$

(where, $\rho$ is density of the component to be measured)
the wet-base exhaust volume (mass) $M(t)$ of the component to be measured can be determined in real time by driving modes as described before. The units used in Eq. (2) are ppm for $C_{DE}(t)$ and cc/min for $M(t)$.

In the modal mass analysis method using this kind of gas trace process, since the moisture content $C_{H2O}$ which is the factor of generating errors as described before in the computation of Eq. (2) is cancelled, it is possible to find the wet-base exhaust volume (mass) $M(t)$ at high accuracy with more simplified computation contents by directly multiplying the dry-base concentration $C_{DE}(t)$ by the dry-base flow rate $Q_{DE}(t)$ found in the same timing by the trace gas detector 8, and the reliability can be remarkably improved. Consequently, particularly in measurement of exhaust gas for low-emission vehicles, big advantages can be achieved in good reproducibility at good sensitivity.

With respect to this point, as described above, because in the conventional CVS method (dilution analysis method), the exhaust gas must be diluted with atmosphere, it is true that the CVS method is not suited for present and future low-emission vehicles. The amount of pollutants emitted from the low-emission vehicles has already been reduced to the level of the amount existing in the atmosphere, and even if the gas is diluted with the atmosphere, in actuality, dilution of exhaust gas is not achieved, and depending on the components, the concentration may rather increase. In addition, in the CVS method, detection by the gas analyzer becomes difficult even if the components are diluted, when the same components are contained already in the undiluted exhaust gas at a low concentration. Even if a large flow-rate air purifier is used to solve this problem, no satisfactory results have been obtained. Under these circumstances, the modal mass analysis method by the trace gas process using this equipment which can accurately measure the exhaust gas flow rate at the end of the tail pipe without diluting it with atmosphere has been proposed.

As described above, according to the exhaust gas analyzer of the invention and the modal mass analysis method by the gas trace process using the exhaust gas analyzer using the analyzer, since no dilution air is used, measurements can be carried out free of influence of specific components contained in the diluting air (atmosphere), free of any compensation of temperature or pressure of exhaust gas, and at the same time, free of pulsation of exhaust gas.

In addition, because the trace gas detector and the gas analyzer are arranged in parallel, the exhaust gas can be introduced into both apparatus in the same timing, the flow rate $Q_{DE}(t)$ of the exhaust gas measured by the trace gas detector and the concentration $C_{DE}(t)$ of the components to be measured by the gas analyzer can be determined in the same timing, and time adjustment between the two apparatus is no longer needed.

In addition, there is an advantage in that the exhaust gas can be sampled from an optional position after the combustion chamber when the trace gas is injected from the suction side of the engine, and from an optional position with a suitable distance provided from the injection point when the trace gas is injected into the tail pipe, and the degree of freedom in the equipment layout can be markedly improved. Because the elimination of CVS equipment and air purifier further increases the degree of freedom, and it is possible to provide the equipment in a compact size and at a low cost. In addition, because the CVS equipment is not used, it has an advantage of requiring no purified air, achieving great reduction in running cost.

Furthermore, in the modal mass analysis method using trace gas by the equipment, because it is possible to actually measure the dry-base flow rate $Q_{DE}(t)$ that can be directly multiplied by the dry-base concentration $C_{DE}(t)$ (see Eq. (1)), it is possible to find the highly accurate measured value at high reliability without using the conventional assumed value of the moisture content in the computation process in Eq. (2).

**Claims**

1. An exhaust gas analyzer for measuring the dry-base concentration ($C_{DE}(t)$) and the dry-base flow rate ($Q_{DE}(t)$) in order to determine the mass ($M(t)$) of specific components in the exhaust gas stream when driven over varying driving modes comprising: a trace gas supply source (10), a flow rate controller (12) for adjusting the supply rate of the trace gas to a vehicle (2), a dehumidifier (5) mounted to a passage (3) of the exhaust gas from the vehicle (2), a trace gas detector (8) and a gas analyzer (7) for measuring a component to be measured in the exhaust gas, **characterized in that** the exhaust gas analyzer includes further two branch supply pipes (14, 15), one (14) to be connected to the suction side of the vehicle and one (15) to be connected to the exhaust gas passage (3), both (7, 8) of trace gas detector (8) and gas analyzer (7) are installed in parallel downstream to the dehumidifier (5) in the passage (3).

2. An exhaust gas analyzer as stated in claim 1, wherein means for computation (17) are incorporated for determining an exhaust volume mass M(t) of the component to be measured by driving modes in accordance with a computation program by means of the modal mass analysis method using the gas trace process set and stored in a memory in advance.

3. A modal mass analysis method comprising the exhaust gas analyzer as stated in claim 1 or claim 2, **characterized in that** a gas trace process is incorporated to find a dry-based exhaust gas flow rate $Q_{DE}(t)$ from a known flow rate $Q_H(t)$ of the trace gas mixed in the exhaust gas emitted from the vehicle (2) offered for a driving simulation test based on a specified driving mode change-over sequence and a concentration $C_H(t)$ of the trace gas measured at a specific sampling time using an arithemetic expression comprised of:

$$Q_{DE}(t) \; = \; Q_H(t) \; \div \; C_H(t) \qquad \qquad \ldots (1)$$

and at the same time to measure the concentration $C_{DE}(t)$ of the component to be measured in the exhaust gas in the specified sampling time which is the same as that for the measurement of the trace gas concentration $C_H(t)$, and to determine the exhaust volume (mass) M(t) of the component to be measured by driving modes by an arithmetic expression comprised of:

$$M(t) \; = \; \rho \; x \; C_{DE}(t) \; x \; Q_{DE}(t) \qquad \qquad \ldots (2)$$

where $\rho$ is density of the component to be measured.

**Patentansprüche**

1. Auspuffgas-Analysevorrichtung zum Messen der trockenbasierten Konzentration ($C_{DE}(t)$) und der trockenbasierten Strömungsrate ($Q_{DE}(t)$), um die Masse (M (t)) spezifischer Komponenten in dem Auspuffgasstrom zu bestimmen, wenn über variierende Fahrmodi gefahren wird, umfassend: eine Spurengas-Zufuhrquelle (10), einen Strömungsraten-Controller (12) zum Einstellen der Zufuhrrate des Spurengases zu einem Fahrzeug (2), einen Entfeuchter (5), der an einem Durchlass (3) des Auspuffgases von dem Fahrzeug (2) angebracht ist, einen Spurengasdetektor (8) und einen Gasanalysator (7) zum Messen einer in dem Auspuffgas zu messenden Komponente, **dadurch gekennzeichnet, dass** die Auspuffgas-Analysevorrichtung ferner zwei Abzweig-Zufuhrrohre (14, 15) einschließt, wobei eines (14) mit der Ansaugseite des Fahrzeugs zu verbinden ist und eines (15) mit dem Auspuffgasdurchlass (3) zu verbinden ist, wobei sowohl der Spurengasdetektor (8) als auch der Gasanalysator (7) parallel stromabwärts des Entfeuchters (5) in dem Durchlass (3) angebracht sind.

2. Auspuffgas-Analysevorrichtung nach Anspruch 1, wobei Berechnungseinrichtungen (17) zum Bestimmen einer Auspuffgas-Volumenmasse M(t) der durch Fahrmodi zu messenden Komponente in Übereinstimmung mit einem Berechnungsprogramm mittels des modalen Masseanalyseverfahrens unter Verwendung des Gasspurprozesses, der im Voraus in einem Speicher eingestellt und gespeichert ist, enthalten sind.

3. Modales Massenanalyseverfahren, umfassend die Auspuffgas-Analysevorrichtung nach Anspruch 1 oder 2, **dadurch gekennzeichnet, dass** ein Gasspurprozess eingeschlossen ist, um eine trockenbasierte Auspuffgas-Strömungsrate $Q_{DE}(t)$ aus einer bekannten Strömungsrate $Q_H(t)$ des Spurengases, das in das Auspuffgas gemischt wird, das aus dem Fahrzeug (2) ausgestoßen wird, das für einen Fahrsimulationstest auf der Grundlage einer spezifizierten Fahrmodus-Wechselsequenz bereitgestellt ist, und eine Konzentration $C_H(t)$ des Spurengases, das bei einer spezifischen Abtastzeit gemessen ist, unter Verwendung eines arithmetischen Ausdrucks, der besteht aus:

$$Q_{DE}(t) \; = \; Q_H(t) \; \div \; C_H(t) \qquad \qquad \ldots (1)$$

zu finden und um gleichzeitig die Konzentration $C_{DE}(t)$ der in dem Auspuffgas zu messenden Komponente in der spezifizierten Abtastzeit zu messen, die die gleiche wie jene für die Messung der Spurengaskonzentration $C_H(t)$ ist, und um das Auspuffgasvolumen (Masse) M(t) der durch Fahrmodi zu messenden Komponente durch einen arithmetischen Ausdruck zu bestimmen, der besteht aus:

$$M(t) = \rho \times C_{DE}(t) \times Q_{DE}(t) \qquad \ldots (2)$$

wobei $\rho$ eine Dichte der zu messenden Komponente ist.

## Revendications

1. Analyseur de gaz d'échappement destiné à mesurer la concentration de base à sec ($C_{DE}(t)$) et le débit de base à sec ($Q_{DE}(t)$) afin de déterminer la masse ($M(t)$) de composants spécifiques dans le flux de gaz d'échappement en conduisant selon des modes de conduite variés comprenant : une source d'alimentation de gaz présent à l'état de trace (10), un régulateur de débit (12) destiné à ajuster le taux d'alimentation à un véhicule du gaz présent à l'état de trace (2), un déshumidificateur (5) monté sur un passage (3) du gaz d'échappement à partir du véhicule (2), un détecteur de gaz présent à l'état de trace (8) et un analyseur de gaz (7) destinés à mesurer un composant devant être mesuré dans le gaz d'échappement, **caractérisé en ce que** l'analyseur de gaz d'échappement comprend en outre deux tuyaux d'alimentation en branche (14, 15), l'un (14) devant être connecté du côté de l'admission du véhicule et l'autre (15) devant être connecté au passage de gaz d'échappement (3), les deux éléments (7, 8) parmi le détecteur de gaz présent à l'état de trace (8) et l'analyseur de gaz (7) sont installés en parallèle en aval du déshumidificateur (5) dans le passage (3).

2. Analyseur de gaz d'échappement selon la revendication 1, dans lequel des moyens de calcul (17) sont incorporés afin de déterminer une masse de volume d'échappement M(t) du composant devant être mesuré par des modes de conduite selon un programme de calcul au moyen de la méthode d'analyse de masse modale utilisant le processus de traçage de gaz défini et stocké dans une mémoire au préalable.

3. Méthode d'analyse de masse modale comprenant l'analyseur de gaz d'échappement selon la revendication 1 ou la revendication 2, **caractérisée en ce qu'**un processus de traçage de gaz est incorporé afin de rechercher un débit de gaz d'échappement de base à sec $Q_{DE}(t)$ à partir d'un débit connu $Q_H(t)$ du gaz présent à l'état de trace mélangé dans le gaz d'échappement émis à partir du véhicule (2) obtenu pour un test de simulation de conduite basé sur une séquence de changement de modes de conduite spécifiée et à partir d'une concentration $C_H(t)$ du gaz présent à l'état de trace mesuré à un temps d'échantillonnage spécifique en utilisant une expression arithmétique constituée de la manière suivante :

$$Q_{DE}(t) = Q_H(t) \div C_H(t) \qquad \ldots (1)$$

et à la fois afin de mesurer la concentration $C_{DE}(t)$ du composant devant être mesuré dans le gaz d'échappement dans la durée d'échantillonnage spécifiée qui est la même que celle pour la mesure de la concentration du gaz présent à l'état de trace $C_H(t)$ et afin de déterminer le volume d'échappement (masse) M(t) du composant devant être mesuré par des modes de conduite par une expression arithmétique constituée de la manière suivante :

$$M(t) = \rho \times C_{DE}(t) \times Q_{DE}(t) \qquad \ldots (2)$$

dans laquelle p est la densité du composant devant être mesuré.

# Fig.1

# Fig.2

Fig. 3